# EUROPÄISCHE PATENTSCHRIFT

(11) **EP 0 842 238 B1**
(45) Veröffentlichungstag und Bekanntmachung des Hinweises auf die Patenterteilung: **21.11.2001**
(21) Anmeldenummer: 96927573.4
(22) Anmeldetag: 22.07.1996
(51) Int. Cl.: C09K 19/30

(54) **FLÜSSIGKRISTALLINES MEDIUM**
LIQUID CRYSTALLINE MEDIUM
MILIEU DE CRISTAL LIQUIDE

(30) Priorität: 01.08.1995 DE 19528105; 14.11.1995 DE 19542285
(43) Veröffentlichungstag der Anmeldung: 20.05.1998
(73) Patentinhaber: MERCK PATENT GmbH, 64293 Darmstadt (DE)
(72) Erfinder: TARUMI, Kazuaki, D-64342 Seeheim (DE); SCHULER, Brigitte, D-63762 Gro ostheim (DE); SCHWARZ, Michael, D-64331 Weiterstadt (DE); ICHINOSE, Hideo, Kanagawa Pref. 250 (JP); NAMIKI, Yasuyoshi, Atsugi-shi, Kanagawa Pref. 243-02 (JP); TAKASHIMA, Akiko, Aikou-gun, Kanagawa Pref. 243-03 (JP); NUMATA, Hiroshi, Yokohama-shi, Kanagawa Pref. 226 (JP)
(86) Internationale Anmeldenummer: EP9603226
(87) Internationale Veröffentlichungsnummer: WO9705214

(56) Entgegenhaltungen:
- EP-A- 0 728 830
- WO-A-91/02043
- WO-A-91/02779
- WO-A-92/06148
- PATENT ABSTRACTS OF JAPAN vol. 9, no. 175 (C-292), 19.Juli 1985 & JP,A,60 045549 (DAINIPPON INK KK)

## Beschreibung

Die vorliegende Erfindung betrifft ein flüssigkristallines Medium, sowie dessen Verwendung für elektrooptische Zwecke und dieses Medium enthaltende Anzeigen.

Flüssige Kristalle werden vor allem als Dielektrika in Anzeigevorrichtungen verwendet, da die optischen Eigenschaften solcher Substanzen durch eine angelegte Spannung beeinflußt werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, daß die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, daß je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfaßt hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei lowvolt-Mischungen war es bisher nicht möglich, sehr hohe spezifische Widerstände zu realisieren. Weiterhin ist es wichtig, daß der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, daß auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebensdauer)

Mit den aus dem Stand der Technik zur Verfügung stehenden Medien ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Der Erfindung liegt die Aufgabe zugrunde, Medien insbesondere für derartige MFK-, TN- oder STN-Anzeigen bereitzustellen, die die oben angegebenen Nachteile nicht oder nur in geringerem Maße, und vorzugsweise gleichzeitig sehr hohe spezifische Widerstände und niedrige Schwellenspannungen aufweisen

Es wurde nun gefunden, daß diese Aufgabe gelöst werden kann, wenn man in Anzeigen erfindungsgemäße Medien verwendet.

Gegenstand der Erfindung ist somit ein flüssigkristallines Medium auf der Basis eines Gemisches von polaren Verbindungen mit positiver dielektrischer Anisotropie, dadurch gekennzeichnet, daß es eine oder mehrere Verbindungen der allgemeinen Formel I 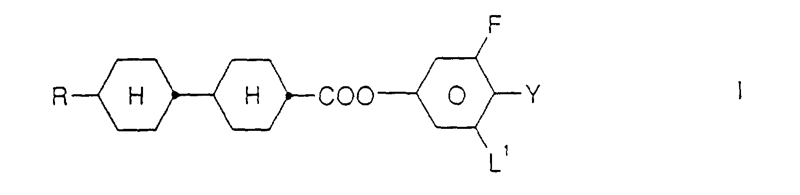 und zusätzlich eine Verbindung der Formel 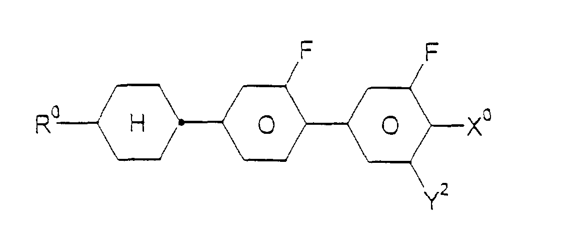 enthält,
worin
- R: H, einen unsubstituierten, einen einfach durch CN oder CF₃ oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S-, 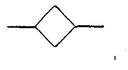 -CO-, -CO-O-, -O-CO- oder -O-CO-O- so ersetzt sein können, daß O-Atome nicht direkt miteinander verknüpft sind,
- Y: F, Cl, halogeniertes Alkyl, Alkenyl oder Alkoxy mit 1 bis 6 C-Atomen, OCH = CF₂, OCF = CF₂, OCF = CFCF₃, OCF = CF-C₂F₅, CF₂OCF₃,
- L¹: H oder F
- R⁰: n-Alkyl, Oxaalkyl, Fluoralkyl oder Alkenyl mit jeweils bis zu 7 C-Atomen,
- X⁰: F, Cl, halogeniertes Alkyl, Alkenyl oder Alkoxy mit 1 bis 6 C-Atomen, OCH = CF₂, OCF = CF₂ und
- Y²: H oder F
bedeuten.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Verbindungen der Formel 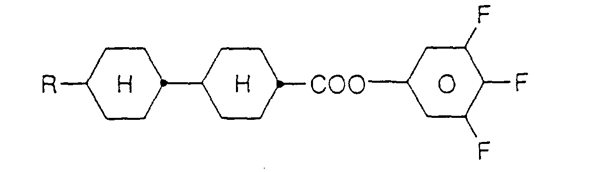 sind bereits aus der EP 0 387 032 bekannt.
Die Verwendung dieser Verbindungen in flüssigkristallinen Mischungen ist aus der EP 0 728 830 A2 (Stand der Technik nach A 54(3), (4) EPÜ für DE und GB) bekannt Das erfindungsgemäße Mischungskonzept wird dort aber nicht offenbart.

In den erfindungsgemäßen Medien enthaltend Verbindungen der Formel I ist Y vorzugsweise F, Cl, OCF₃, OCHF₂, CF₃, CHFCF₃, CF₂CHF₂, C₂H₄CHF₂, CF₂CH₂CF₃,CHF₂, OCH₂CF₃, OCH₂CHF₂, OCF₂CHF₂, O(CH₂)₃CF₃, OCH₂C₂F₅, OCH₂CF₂CHF₂, OCH₂C₃F₇, OCHFCF₃, OC₂F₅, OCF₂CHFCF₃, OCH=CF₂, OCF=CF₂, OCF=CFCF₃, OCF=CF-C₂F₅, CH=CHF, CH=CF₂, CF=CF₂, CF₂OCF₃, insbesondere F, OCHFCF₃, OCF₃, OCHF₂, OC₂F₅, OC₃F₇, OCH=CF₂, und CF₂OCF₃.

Insbesondere bevorzugt sind Medien enthaltend Verbindungen der Formel I, worin Y = L¹ = F bedeuten.

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R einen einfach durch CN oder CF₃ substituierten Alkyl- oder Alkenylrest bedeutet, so ist dieser Rest vorzugsweise geradkettig. Die Substitution durch CN oder CF₃ ist in beliebiger Position.

Falls R einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I, die über für Polymerisationsreaktionen geeignete Flügelgruppen R verfügen, eignen sich zur Darstellung flüssigkristalliner Polymerer.

Verbindungen der Formel I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxyethyl, 3,3-Bis-carboxy-propyl, 4,4-Bis-carboxy-butyl, 5,5-Bis-carboxypentyl, 6,6-Bis-carboxy-hexyl, 7,7-Bis-carboxy-heptyl, 8,8-Bis-carboxyoctyl, 9,9-Bis-carboxy-nonyl, 10,10-Bis-carboxy-decyl, Bis(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel I können z.B. wie folgt hergestellt werden:

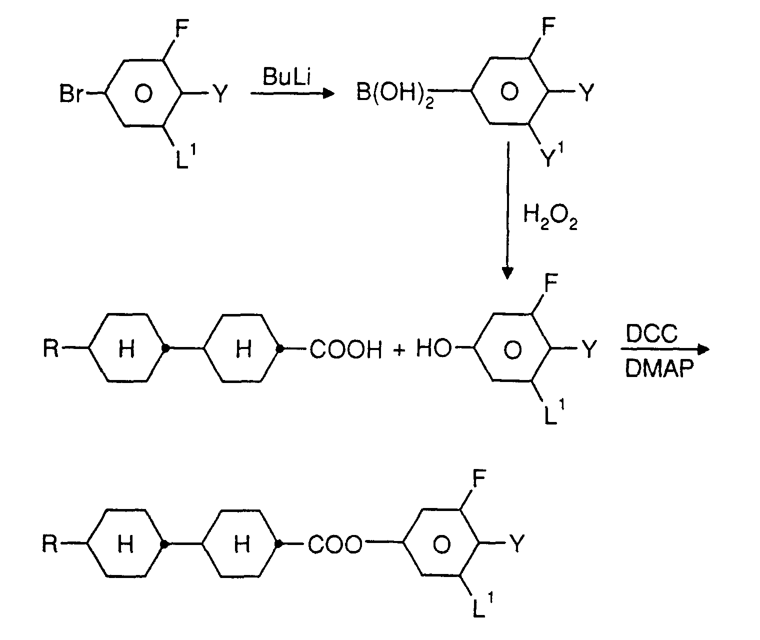

Gegenstand der Erfindung sind auch elektrooptische Anzeigen (insbesondere STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes.

Die erzielbaren Kombinationen aus Klärpunkt, Viskosität bei tiefer Temperatur, thermischer und UV-Stabilität und dielektrischer Anisotropie übertreffen bei weitem bisherige Materialien aus dem Stand der Technik.

Die Forderung nach hohem Klärpunkt, nematischer Phase bei tiefer Temperatur sowie einem hohen Δε konnte bislang nur unzureichend erfüllt werden. Systeme wie z.B. ZLI-3119 weisen zwar vergleichbaren Klärpunkt und vergleichbar günstige Viskositäten auf, besitzen jedoch ein Δε von nur +3.

Andere Mischungs-Systeme besitzen vergleichbare Viskositäten und Werte von Δε, weisen jedoch nur Klärpunkte in der Gegend von 60 °C auf.

Die erfindungsgemäßen Flüssigkristallmischungen ermöglichen es bei Beibehaltung der nematischen Phase bis -20 °C und bevorzugt bis -30 °C, besonders bevorzugt bis -40 °C, Klärpunkte oberhalb 80°, vorzugsweise oberhalb 90°, besonders bevorzugt oberhalb 100 °C, gleichzeitig dielektrische Anisotropiewerte Δε ≥ 6, vorzugsweise ≥ 8 und einen hohen Wert für den spezifischen Widerstand zu erreichen, wodurch hervorragende STN- und MKF-Anzeigen erzielt werden können. Insbesondere sind die Mischungen durch kleine Operationsspannungen gekennzeichnet. Die TN-Schwellen liegen unterhalb 2,0 V, vorzugsweise unterhalb 1,5 V, besonders bevorzugt < 1,3 V.

Es versteht sich, daß durch geeignete Wahl der Komponenten der erfindungsgemäßen Mischungen auch höhere Klärpunkte (z.B. oberhalb 110°) bei höheren Schwellenspannung oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringeren Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum eine kleinerere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Die Viskosität bei 20 °C ist vorzugsweise < 60 mPa.s, besonders bevorzugt < 50 mPa.s. Der nematische Phasenbereich ist vorzugsweise mindestens 90°, insbesondere mindestens 100°. Vorzugsweise erstreckt sich dieser Bereich mindestens von -20° bis +80°.

Messungen des "Capacity Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, daß erfindungsgemäße Mischungen enthaltend Verbindungen der Formel I eine deutlich kleinere Abnahme des HR mit steigender Temperatur aufweisen als analoge Mischungen enthaltend anstelle den Verbindungen der Formel I Cyanophenylcyclohexane der 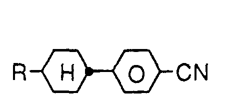 oder Ester der Formel Formel

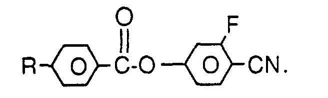

Auch die UV-Stabilität der erfindungsgemäßen Mischungen ist erheblich besser, d. h. sie zeigen eine deutlich kleinere Abnahme des HR unter UV-Belastung.

Vorzugsweise basieren die erfindungsgemäßen Medien auf mehreren (vorzugsweise zwei oder mehr) Verbindungen der Formel I, d.h. der Anteil dieser Verbindungen ist 5-95 %, vorzugsweise 10-60 % und besonders bevorzugt im Bereich von 20-50 %.

Die einzelnen Verbindungen der Formeln I bis XII und deren Unterformeln, die in den erfindungsgemäßen Medien verwendet werden können, sind entweder bekannt, oder sie können analog zu den bekannten Verbindungen hergestellt werden.

Bevorzugte Ausführungsformen sind im folgenden angegeben:
- Medium enthält zusätzlich eine oder mehrere Verbindungen ausgewählt aus der Gruppe bestehend aus den allgemeinen Formeln II bis VI: 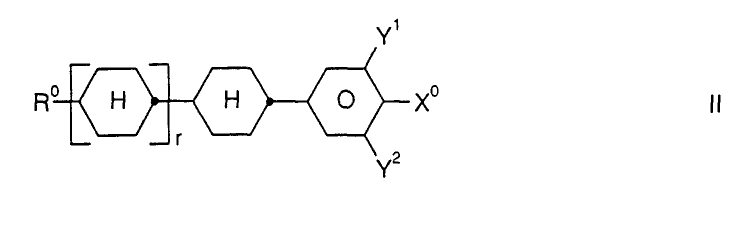 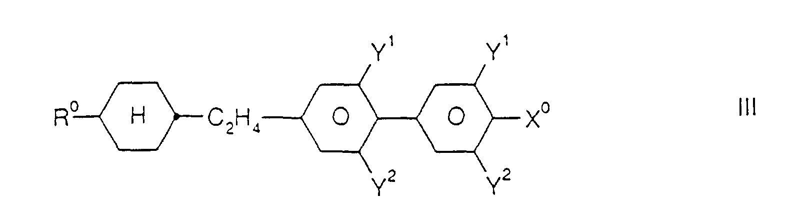 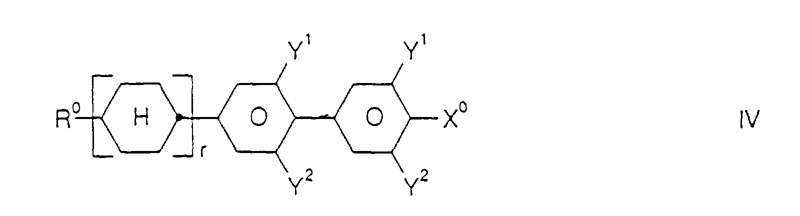 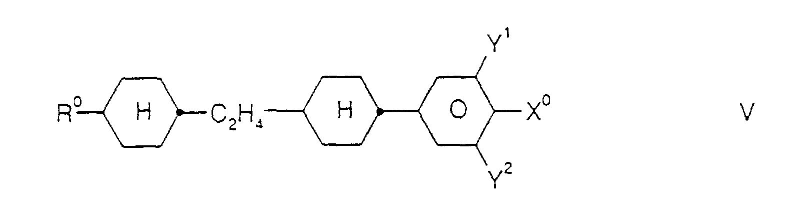 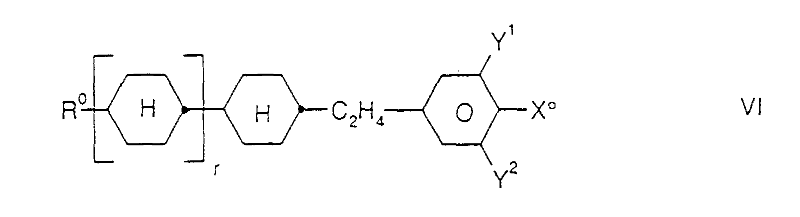 worin die einzelnen Reste die folgenden Bedeutungen haben:
   - R⁰:: n-Alkyl, Oxaalkyl, Fluoralkyl oder Alkenyl mit jeweils bis zu 9 C-Atomen
   - X⁰:: F, Cl, halogeniertes Alkyl, Alkenyl oder Alkoxy mit 1 bis 6 C-Atomen, OCH = CF₂, OCF = CF₂
   - Y¹ und Y²:: jeweils unabhängig voneinander H oder F
   - r:: 0 oder 1,

Die Verbindung der Formel IV ist vorzugsweise 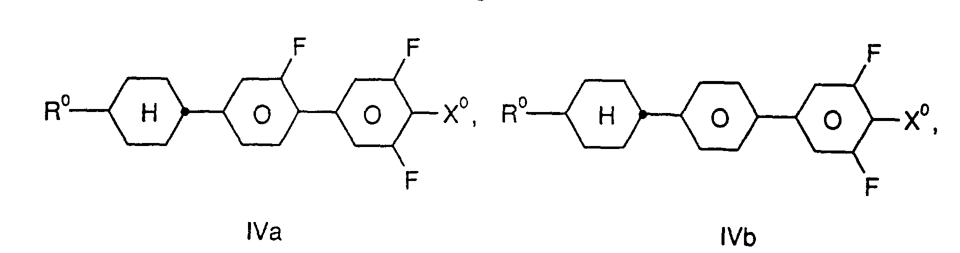 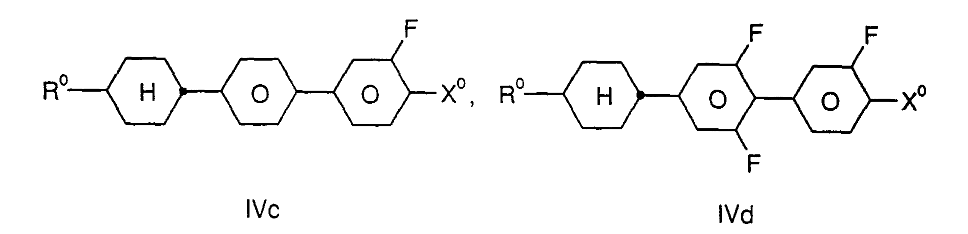 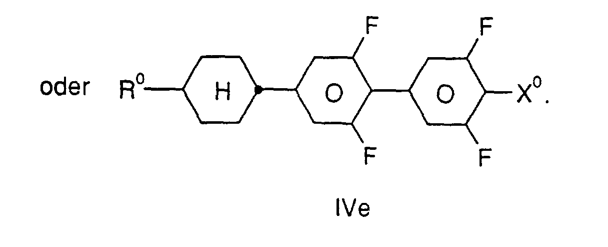
- Medium enthält zusätzlich eine oder mehrere Verbindungen ausgewählt aus der Gruppe bestehend aus den allgemeinen Formeln VII bis XII: 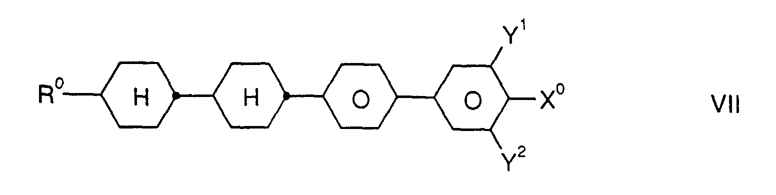 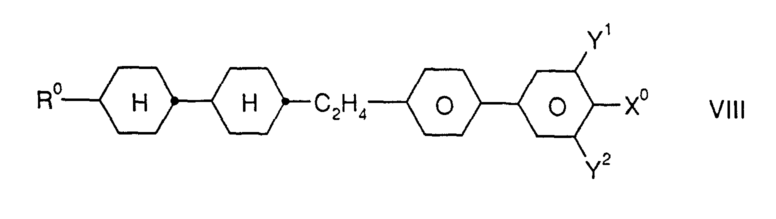 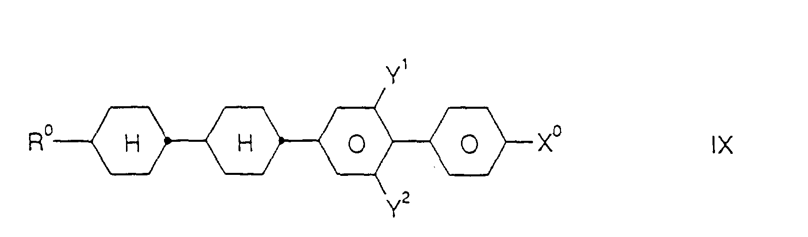 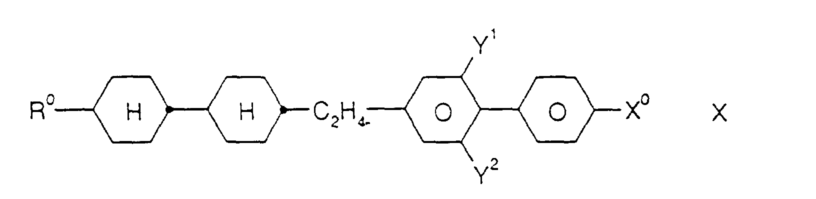 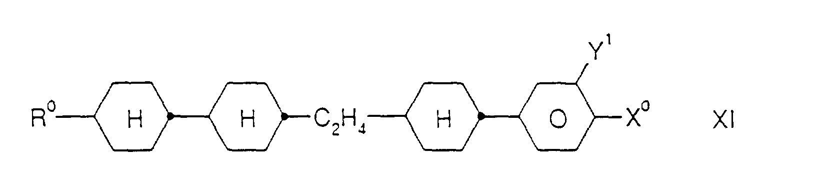 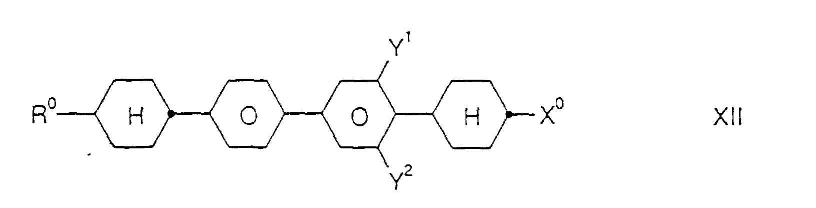
worin R⁰, X⁰, Y¹ und Y² jeweils unabhängig voneinander eine der in Anspruch 2 angegebene Bedeutung haben.
- der Anteil an Verbindungen der Formeln I bis VI zusammen beträgt im Gesamtgemisch mindestens 50 Gew.-%;
- der Anteil an Verbindungen der Formel I beträgt im Gesamtgemisch 10 bis 50 Gew.-%;
- der Anteil an Verbindungen der Formeln II bis VI im Gesamtgemisch beträgt 20 bis 80 Gew.-% 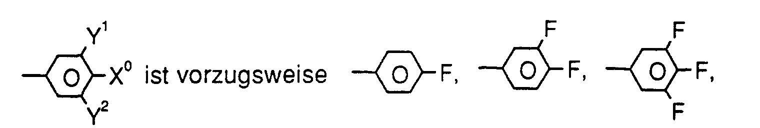 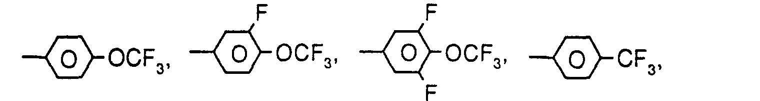 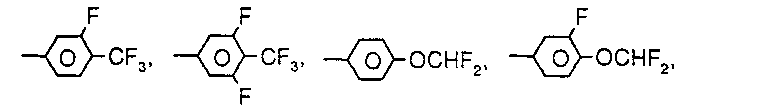 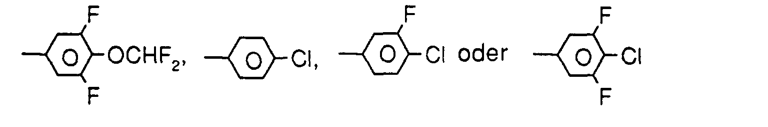
- das Medium enthält Verbindungen der Formeln II, III, IV, V und/ oder VI
- Medium enthält zusätzlich ein oder mehrere Verbindungen der Formeln 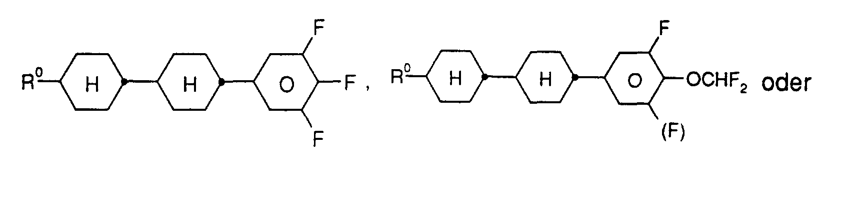 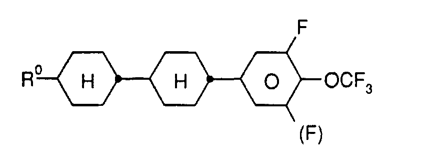
- R⁰ ist geradkettiges Alkyl oder Alkenyl mit 2 bis 7 C-Atomen
- das Medium besteht im wesentlichen aus Verbindungen der Formeln I bis VI
- das Medium enthält zusätzlich ein oder mehrere Verbindungen der Formel XVII 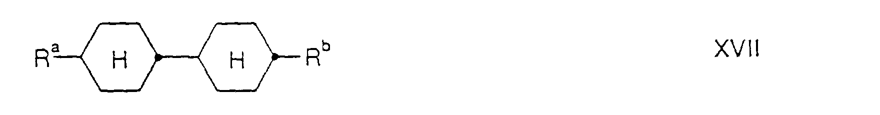 worin R^{a} und R^{b} jeweils unabhängig voneinander geradkettiges Alkyl oder Alkoxy mit 1 bis 5 C-Atomen bedeuten
- das Medium besteht im wesentlichen aus Verbindungen der Formeln I bis VI und XVII
- das Medium enthält weitere Verbindungen, vorzugsweise ausgewählt aus der folgenden Gruppe bestehend aus den aligemeinen Formeln XIII bis XVI: 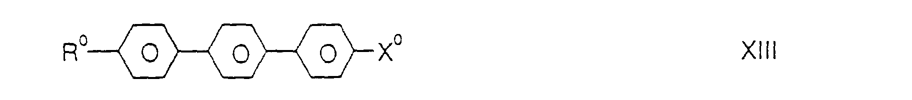 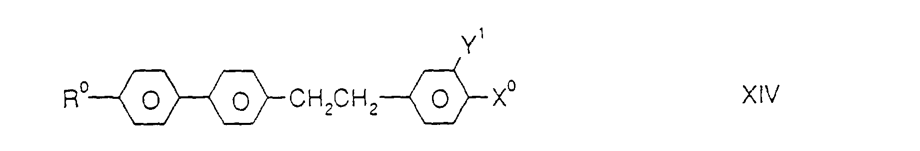 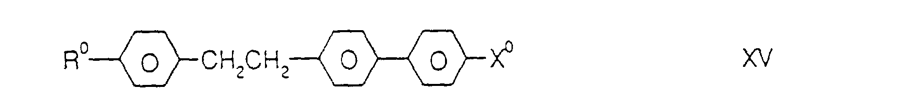 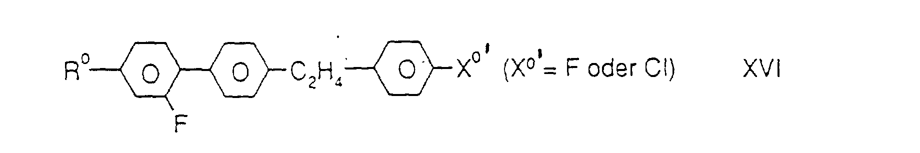 worin R⁰ und X⁰ die oben angegebene Bedeutung haben und die 1,4-Phenylenringe durch CN, Chlor oder Fluor substituiert sein können. Vorzugsweise sind die 1,4-Phenylenringe ein- oder mehrfach durch Fluoratome substituiert.
- Das Gewichtsverhältnis I: (II + III + IV + V + VI) ist vorzugsweise 1 : 10 bis 10 : 1.
- Medium besteht im wesentlichen aus Verbindungen ausgewählt aus der Gruppe bestehend aus den allgemeinen Formeln I bis XII.

Es wurde gefunden, daß bereits ein relativ geringer Anteil an Verbindungen der Formel I im Gemisch mit üblichen Flüssigkristallmaterialien, insbesondere jedoch mit einer oder mehreren Verbindungen der Formel II, III, IV, V und/oder VI zu einer beträchtlichen Erniedrigung der Schwellenspannung und zu niedrigen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Bevorzugt sind insbesondere Mischungen, die neben ein oder mehrerer Verbindungen der Formel I ein oder mehrere Verbindungen der Formel IV enthalten, insbesondere Verbindungen der Formel IVa, worin X⁰ F oder OCF₃ bedeutet. Die Verbindungen der Formeln I bis VI sind farblos, stabil und untereinander und mit anderen Flüssigkristallmaterialien gut mischbar.

Der Ausdruck "Alkyl" umfaßt geradkettige und verzweigte Alkylgruppen mit 1-7 Kohlenstoffatomen, insbesondere die geradkettigen Gruppen Methyl, Ethyl, Propyl, Butyl, Pentyl, Hexyl und Heptyl. Gruppen mit 2-5 Kohlenstoffatomen sind im allgemeinen bevorzugt.

Der Ausdruck "Alkenyl" umfaßt geradkettige und verzweigte Alkenylgruppen mit 2-7 Kohlenstoffatomen, insbesondere die geradkettigen Gruppen. Besonders Alkenylgruppen sind C₂₋C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfaßt vorzugsweise geradkettige Gruppen mit endständigen Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" umfaßt vorzugsweise geradkettige Reste der Formel CₙH₂ₙ₊₁-O-(CH₂)ₘ, worin n und m jeweils unabhängig voneinander 1 bis 6 bedeuten. Vorzugsweise ist n = 1 und m 1 bis 6.

Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1 E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten.

Eine -CH₂CH₂-Gruppe führt im allgemeinen zu höheren Werten von k₃₃/k₁₁ im Vergleich zu einer einfachen Kovalenzbindung. Höhere Werte von k₃₃/k₁₁ ermöglichen z.B. flachere Transmissionskennlinien in TN-Zellen mit 90° Verdrillung (zur Erzielung von Grautönen) und steilere Transmissionskennlinien in STN-, SBE- und OMI-Zellen (höhere Multiplexierbarkeit) und umgekehrt.

Das optimale Mengenverhältnis der Verbindungen der Formeln I und II + III + IV + V + VI hängt weitgehend von den gewünschten Eigenschaften, von der Wahl der Komponenten der Formeln I, II, III, IV, V und/oder VI und von der Wahl weiterer gegebenenfalls vorhandener Komponenten ab. Geeignete Mengenverhältnisse innerhalb des oben angegebenen Bereichs können von Fall zu Fall leicht ermittelt werden.

Die Gesamtmenge an Verbindungen der Formeln I bis XII in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln I bis XII ist.

In einer besonders bevorzugten Ausführungsform enthalten die erfindungsgemäßen Medien Verbindungen der Formel II bis VI (vorzugsweise II, III und/oder IV, insbesondere IVa), worin X⁰ F, OCF₃, OCHF₂, F, OCH=CF₂, OCF=CF₂ oder OCF₂-CF₂H bedeutet. Eine günstige synergistische Wirkung mit den Verbindungen der Formel I führt zu besonders vorteilhaften Eigenschaften. Insbesondere Mischungen enthaltend Verbindungen der Formel I und der Formel IVa zeichnen sich durch ihre niedrigen Schwellenspannungen aus.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefaßt und umfaßt auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze enthalten. Beispielsweise können 0-15 % pleochroitische Farbstoffe oder chirale Dotierstoffe zugesetzt werden.

C bedeutet eine kristalline, S eine smektische, S_{C} eine smektisch C, N eine nematische und I die isotrope Phase.

V₁₀ bezeichnet die Spannung für 10 % Transmission (Blickrichtung senkrecht zur Plattenoberfläche). tₒₙ bezeichnet die Einschaltzeit und t_{off} die Ausschaltzeit bei einer Betriebsspannung entsprechend dem 2,5fachen Wert von V₁₀. Δn bezeichnet die optische Anisotropie und nₒ den Brechungsindex. Δε bezeichnet die dielektrische Anisotropie (Δε = ε_{∥} - ε_{⊥}, wobei ε_{∥} die Dielektrizitätskonstante parallel zu den Moleküllängsachsen und ε_{⊥} die Dielektrizitätskonstante senkrecht dazu bedeutet). Die elektrooptischen Daten wurden in einer TN-Zelle im 1. Minimum (d.h. bei einem d Δn-Wert von 0,5) bei 20 °C gemessen, sofern nicht ausdrücklich etwas anderes angegeben wird. Die optischen Daten wurden bei 20 °C gemessen, sofern nicht ausdrücklich etwas anderes angegeben wird.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nF.F.F | CₙH₂ₙ₊₁ | F | F | F |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nAm | CₙH₂ₙ₊₁ | COOCₘH₂ₘ₊₁ | H | H |
| nOCCF₂.F.F | CₙH₂ₙ₊₁ | OCH₂CF₂H | F | F |

Bevorzugte Mischungskomponenten finden sich in den Tabellen A und B.

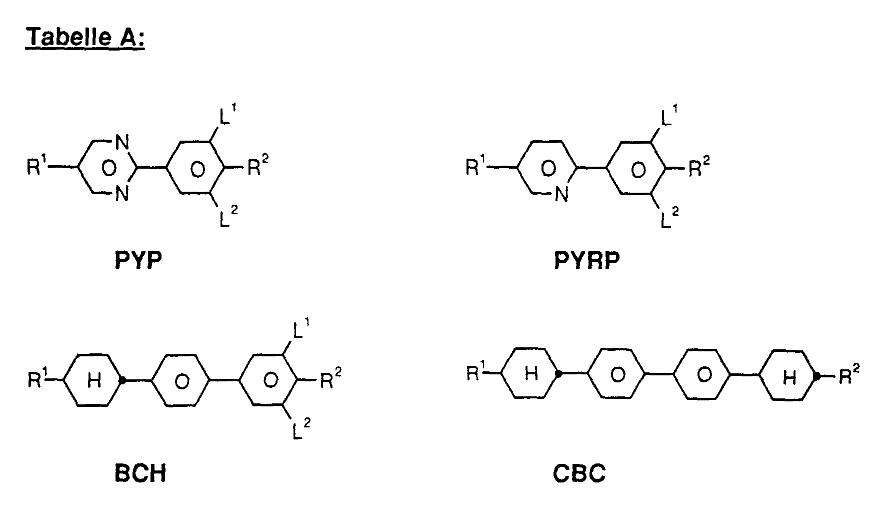

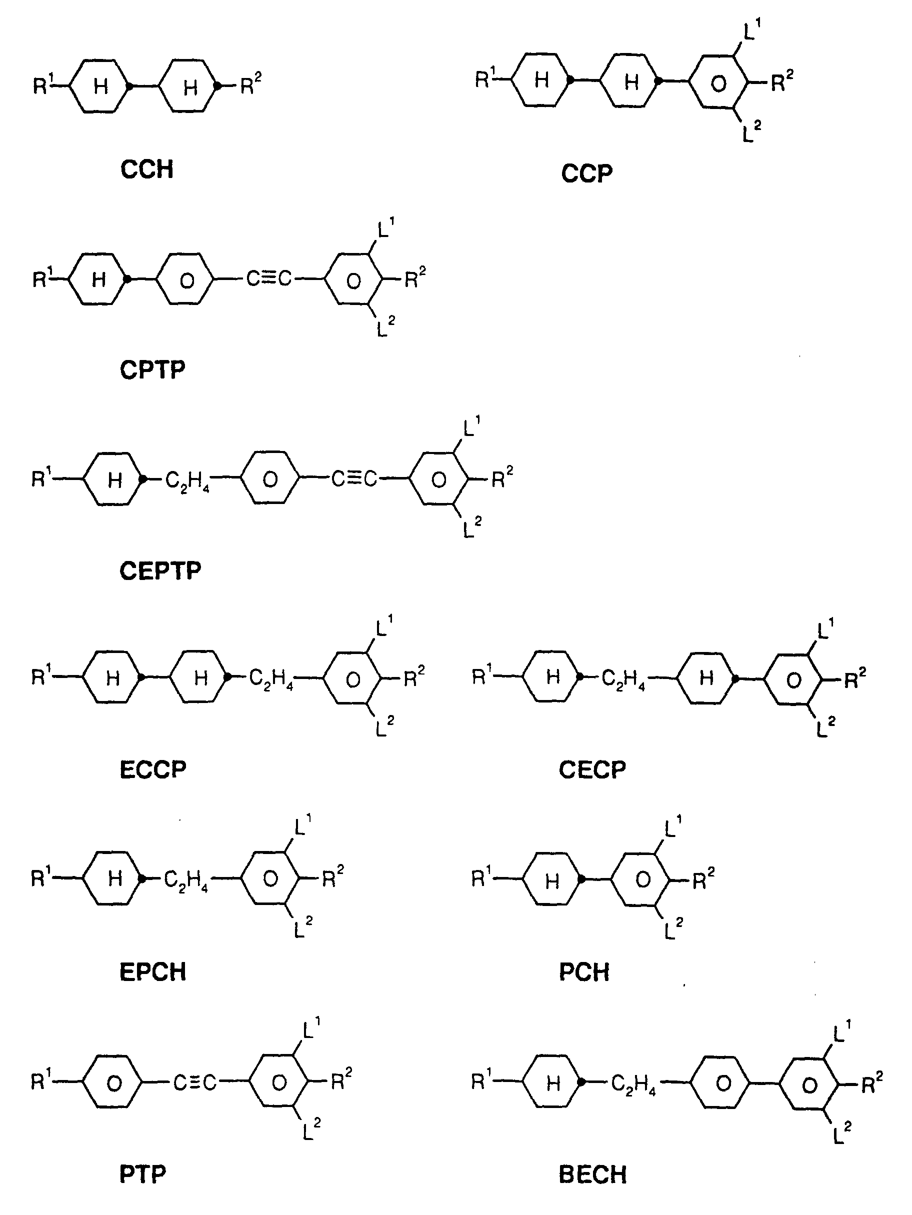

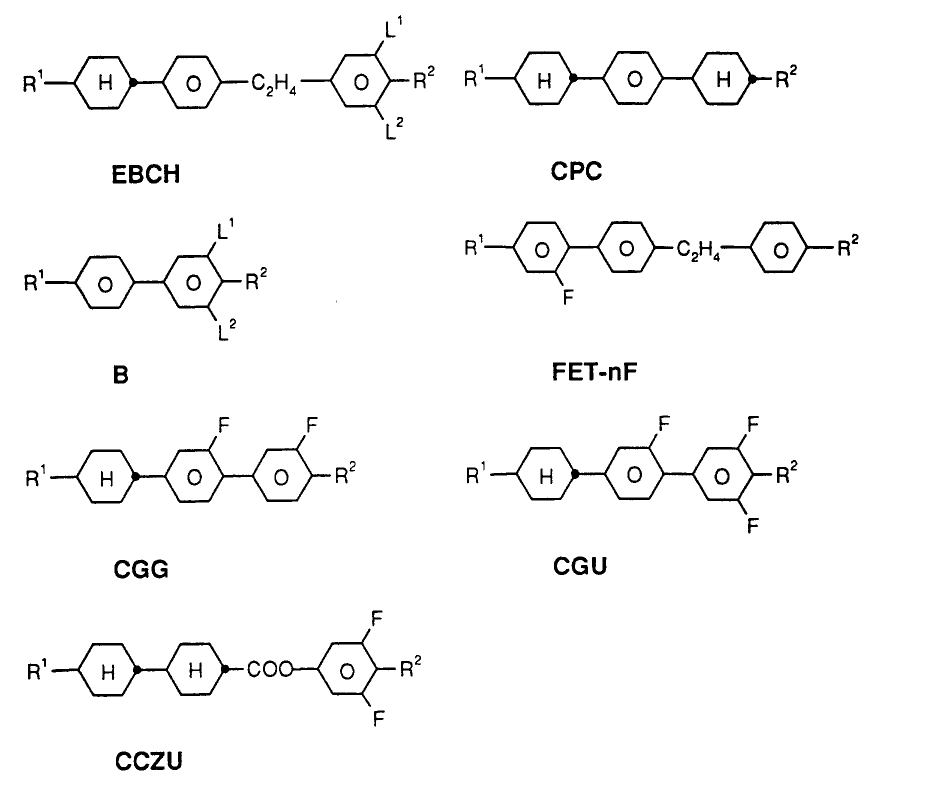

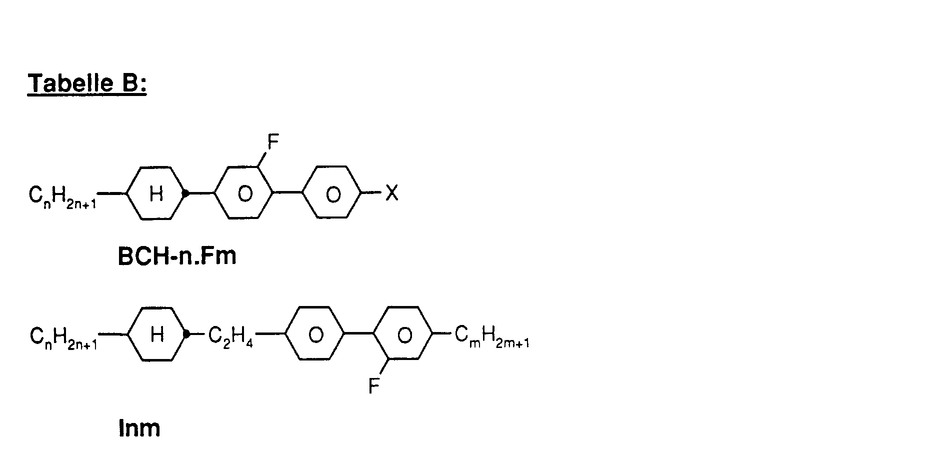

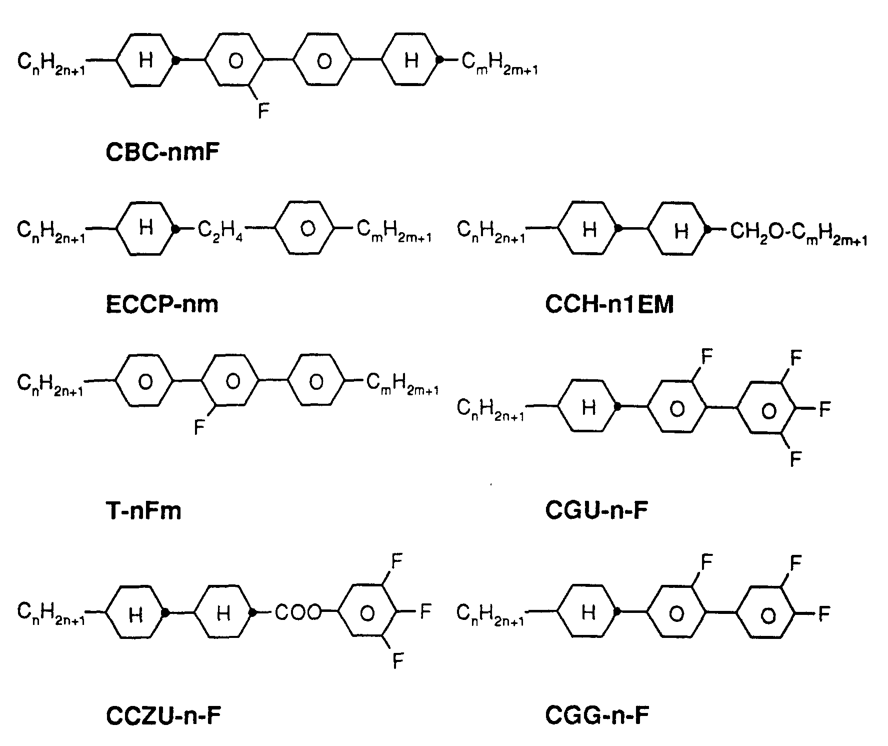

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Dichlormethan, Diethylether, Methyl-tert.Butylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DCC: Dicyclohexylcarbodiimid
- DMAP: Dimethylaminopyridin
- DMEU: 1,3-Dimethyl-2-imidazolidinon
- KOT: Kalium-tertiär-butanolat
- THF: Tetrahydrofuran
- pTsOH: p-Toluolsulfonsäure

### Beispiel 1

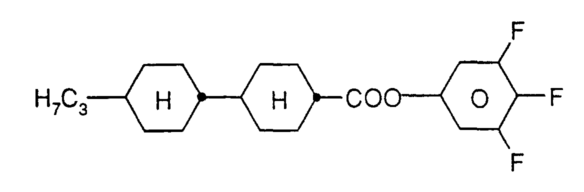

80 mmol trans-4-(4-Propylcyclohexyl)cyclohexansäure, 0,1 mol 3,4,5-Trifluorphenol und 8,8 mmol DMAP werden in 460 g Dichlormethan gelöst und mit 88 mmol DCC versetzt. Die Mischung wird über Nacht bei Raumtemperatur gerührt. Der ausgefallene Dicyclohexylharnstoff wird abgesaugt und das Filtrat zum Rückstand einrotiert. Das Rohprodukt wird mit Toluol über eine Kieselgelsäule chromatographiert. Das Filtrat wird eingeengt und der Rückstand wird aus n-Hexan umkristallisiert. K 56 N 117,2 I; Δn = 0,070; Δε = 11,33

Analog werden die folgenden Verbindungen der Formel 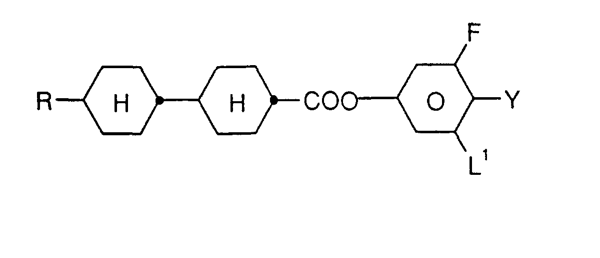 hergestellt:

| R¹ | Y | L¹ | |
|---|---|---|---|
| CH₃ | F | H | |
| CH₃ | F | F | |
| C₂H₅ | F | H | |
| C₂H₅ | F | F | K 80 N 81,3 I; Δn = +0,061; Δε = 11,02 |
| n-C₃H₇ | F | H | |
| n-C₄H₉ | F | H | |
| n-C₄H₉ | F | F | K 73 N 115,7 I; Δn = +0,069; Δε = 10,87 |
| n-C₅H₁₁ | F | H | |
| n-C₅H₁₁ | F | F | K 72 N 123,1 I; Δn = +0,070; Δε = 10,71 |
| n-C₆H₁₃ | F | H | |
| n-C₆H₁₃ | F | F | |
| | | | |
| CH₃ | OCF₃ | H | |
| CH₃ | OCF₃ | F | |
| C₂H₅ | OCF₃ | H | |
| C₂H₅ | OCF₃ | F | |
| n-C₃H₇ | OCF₃ | H | |
| n-C₃H₇ | OCF₃ | F | |
| n-C₅H₁₁ | OCF₃ | H | |
| n-C₅H₁₁ | OCF₃ | F | |
| n-C₆H₁₃ | OCF₃ | H | |
| n-C₆H₁₃ | OCF₃ | F | |
| | | | |
| CH₃ | OCHF₂ | H | |
| CH₃ | OCHF₂ | F | |
| C₂H₅ | OCHF₂ | H | |
| C₂H₅ | OCHF₂ | F | |
| n-C₃H₇ | OCHF₂ | H | |

| | | |
|---|---|---|
| n-C₃H₇ | OCHF₂ | F |
| n-C₅H₁₁ | OCHF₂ | H |
| n-C₅H₁₁ | OCHF₂ | F |
| n-C₆H₁₃ | OCHF₂ | H |
| n-C₆H₁₃ | OCHF₂ | F |
| | | |
| CH₃ | Cl | H |
| CH₃ | Cl | F |
| C₂H₅ | Cl | H |
| C₂H₅ | Cl | F |
| n-C₃H₇ | Cl | H |
| n-C₃H₇ | Cl | F |
| n-C₅H₁₁ | Cl | H |
| n-C₅H₁₁ | Cl | F |
| n-C₆H₁₃ | Cl | H |
| n-C₆H₁₃ | Cl | F |
| | | |
| CH₃ | OCHFCF₃ | H |
| CH₃ | OCHFCF₃ | F |
| C₂H₅ | OCHFCF₃ | H |
| C₂H₅ | OCHFCF₃ | F |
| n-C₃H₇ | OCHFCF₃ | H |
| n-C₃H₇ | OCHFCF₃ | F |
| n-C₅H₁₁ | OCHFCF₃ | H |
| n-C₅H₁₁ | OCHFCF₃ | F |
| n-C₆H₁₃ | OCHFCF₃ | H |
| n-C₆H₁₃ | OCHFCF₃ | F |

| | | |
|---|---|---|
| CH₃ | OCH = CF₂ | H |
| CH₃ | OCH = CF₂ | F |
| C₂H₅ | OCH = CF₂ | H |
| C₂H₅ | OCH = CF₂ | F |
| n-C₃H₇ | OCH = CF₂ | H |
| n-C₃H₇ | OCH = CF₂ | F |
| n-C₅H₁₁ | OCH = CF₂ | H |
| n-C₅H₁₁ | OCH = CF₂ | F |
| n-C₆H₁₃ | OCH = CF₂ | H |
| n-C₆H₁₃ | OCH = CF₂ | F |
| | | |
| CH₃ | OC₂F₅ | H |
| CH₃ | OC₂F₅ | F |
| C₂H₅ | OC₂F₅ | H |
| C₂H₅ | OC₂F₅ | F |
| n-C₃H₇ | OC₂F₅ | H |
| n-C₃H₇ | OC₂F₅ | F |
| n-C₅H₁₁ | OC₂F₅ | H |
| n-C₅H₁₁ | OC₂F₅ | F |
| n-C₆H₁₃ | OC₂F₅ | H |
| n-C₆H₁₃ | OC₂F₅ | F |

### Mischungsbeispiele

### Beispiel A

| | | | |
|---|---|---|---|
| CCP-2F.F.F | 13,0 % | Klärpunkt [°C] | 70 |
| CCP-3F.F.F | 9,0 % | Δn [589 nm, 20 °C] | +0,0885 |
| CCP-5F.F.F | 7,0 % | Δε [1 kHz, 20 °C] | 12,9 |
| CCP-3OCF₃ | 8,0 % | V_{(10,0,20)} [V] | 0,87 |
| CGU-2-F | 14,0 % | | |
| CGU-3-F | 11,0 % | | |
| CGU-5-F | 8,0 % | | |
| CCZU-3-F | 20,0 % | | |
| CCZU-5-F | 10,0 % | | |

### Beispiel B

| | | | |
|---|---|---|---|
| CCP-2F.F.F | 10,0 % | Klärpunkt [°C] | 82 |
| CCP-3F.F.F | 9,0 % | Δn [589 nm, 20 °C] | +0,0910 |
| CCP-5F.F.F | 5,0 % | Δε [1 kHz, 20 °C] | 12,6 |
| CCP-3OCF₃ | 9,0 % | V_{(10,0,20)} [V] | 1,02 |
| CCP-5OCF₃ | 8,0 % | | |
| CGU-2-F | 12,0 % | | |
| CGU-3-F | 11,0 % | | |
| CGU-5-F | 6,0 % | | |
| CCZU-3-F | 20,0 % | | |
| CCZU-5-F | 10,0 % | | |

### Beispiel C

| | | | |
|---|---|---|---|
| CCP-2F.F.F | 11,0 % | Klärpunkt [°C] | +70 |
| CCP-3F.F.F | 10,0 % | Δn [589 nm, 20 °C] | +0,0879 |
| CCP-5F.F.F | 6,0 % | Δε [1 kHz, 20 °C] | 12,5 |
| CCP-3OCF₃ | 6,0 % | V_{(10,0,20)} [V] | 1,17 |
| CCP-5OCF₃ | 4,0 % | (V₉₀/V₁₀ -1) 100 [%] | 69,1 |
| CGU-2-F | 13,0 % | | |
| CGU-3-F | 12,0 % | | |
| CGU-5-F | 8,0 % | | |
| CCZU-2-F | 7,0 % | | |
| CCZU-3-F | 15,0 % | | |
| CCZU-5-F | 8,0 % | | |

### Beispiel D

| | | | |
|---|---|---|---|
| CCP-3F.F.F | 13,0 % | Klärpunkt [°C] | +84 |
| CCP-5F.F.F | 8,0 % | Δn [589 nm, 20 °C] | +0,0922 |
| CCP-3OCF₃ | 8,0 % | V_{(10,0,20)} [V] | 1,34 |
| CCP-5OCF₃ | 8,0 % | (V₉₀/V₁₀ -1) 100 [%] | 63,6 |
| CCP-3OCF₂.F.F | 3,0 % | | |
| CGU-2-F | 10,0 % | | |
| CGU-3-F | 12,0 % | | |
| CGU-5-F | 8,0 % | | |
| CCZU-2-F | 7,0 % | | |
| CCZU-3-F | 15,0 % | | |
| CCZU-5-F | 8,0 % | | |

### Beispiel E

| | | | |
|---|---|---|---|
| CCP-2F.F.F | 12,0 % | Klärpunkt [°C] | +88 |
| CCP-3F.F.F | 13,0 % | Δn [589 nm, 20 °C] | +0,0830 |
| CCP-5F.F.F | 8,0 % | V_{(10,0,20)} [V] | 1,44 |
| CCP-2OCF₂.F.F | 4,0 % | (V₉₀/V₁₀ -1)· 100 [%] | 64,44 |
| CCP-2OCF₃ | 8,0 % | | |
| CCP-3OCF₃ | 8,0 % | | |
| CCP-5OCF₃ | 4,0 % | | |
| CGU-2-F | 8,0 % | | |
| CGU-3-F | 5,0 % | | |
| CCZU-2-F | 7,0 % | | |
| CCZU-3-F | 16,0 % | | |
| CCZU-5-F | 7,0 % | | |

### Beispiel F

| | | | |
|---|---|---|---|
| CCP-2F.F.F | 10,0 % | Klärpunkt [°C] | +77 |
| CCP-3F.F.F | 10,0 % | Δn [589 nm, 20 °C] | +0,0893 |
| CCP-5F.F.F | 4,0 % | V_{(10,0,20)} [V] | 1,03 |
| CCP-3OCF₃ | 8,0 % | (V₉₀/V₁₀ -1) 100 [%] | 61,8 |
| CCP-4OCF₃ | 5,0 % | | |
| CCP-5OCF₃ | 3,0 % | | |
| CGU-2-F | 12,0 % | | |
| CGU-3-F | 12,0 % | | |
| CGU-5-F | 6,0 % | | |
| CCZU-2-F | 7,0 % | | |
| CCZU-3-F | 16,0 % | | |
| CCZU-5-F | 7,0 % | | |

### Beispiel G

| | | | |
|---|---|---|---|
| CCP-2F.F.F | 3,0 % | Klärpunkt [°C] | +75 |
| CCP-3F.F.F | 12,0 % | Δn [589 nm, 20 °C] | +0,0947 |
| CCP-3OCF₃ | 8,0 % | Δε [1 kHz, 20 °C] | 12,1 |
| CCP-5OCF₃ | 8,0 % | V_{(10,0,20)} [V] | 0,99 |
| CGU-2-F | 12,0 % | (V₉₀/V₁₀ -1). 100 [%] | 61,6 |
| CGU-3-F | 12,0 % | | |
| CGU-5-F | 7,0 % | | |
| CCZU-2-F | 7,0 % | | |
| CCZU-3-F | 16,0 % | | |
| CCZU-5-F | 7,0 % | | |
| BCH-2F.F | 8,0 % | | |

### Beispiel H

| | | | |
|---|---|---|---|
| PCH-7F | 1,5 % | Klärpunkt [°C] | +77 |
| CCP-2F.F.F | 8,0 % | Δn [589 nm, 20 °C] | +0,0861 |
| CCP-3F.F.F | 10,0% | Δε [1 kHz, 20°C] | +11,2 |
| CCP-5F.F.F | 5,0 % | V_{(10,0,20)} [V] | 1,17 |
| CCP-2OCF₃ | 9,0 % | | |
| CCP-3OCF₃ | 6,0 % | | |
| CCP-5OCF₃ | 4,0 % | | |
| CGU-2-F | 9,0 % | | |
| CGU-3-F | 8,5 % | | |
| CGU-5-F | 10,0 % | | |
| CCH-35 | 6,0 % | | |
| CCZU-2-F | 3,0 % | | |
| CCZU-3-F | 18,0 % | | |
| CCZU-5-F | 2,0 % | | |

### Beispiel I

| | | | |
|---|---|---|---|
| CCH-35 | 6,0 % | Klärpunkt [°C] | +82 |
| CCP-2OCF₂.F.F | 11,0 % | Δn [589 nm, 20 °C] | +0,0824 |
| CCP-2F.F.F | 12,0% | Δε[1 kHz, 20 °C] | +10,4 |
| CCP-3OCF₃ | 8,0 % | V_{(10,0,20)} [V] | 1,16 |
| CCP-3F.F.F | 11,0 % | | |
| CCP-5OCF₃ | 8,0 % | | |
| CCP-5F.F.F | 8,0 % | | |
| CCZU-2-F | 8,0 % | | |
| CCZU-3-F | 12,0 % | | |
| CGU-2-F | 10,0 % | | |
| CGU-3-F | 6,0 % | | |

## Patentansprüche

1. Flüssigkristallines Medium auf der Basis eines Gemisches von polaren Verbindungen mit positiver dielektrischer Anisotropie, **dadurch gekennzeichnet, daß** es eine oder mehrere Verbindungen der allgemeinen Formel I 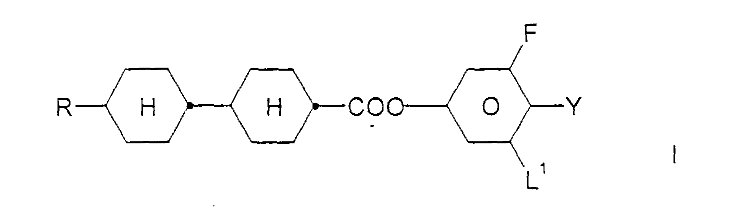 und zusätzlich eine Verbindung der Formel 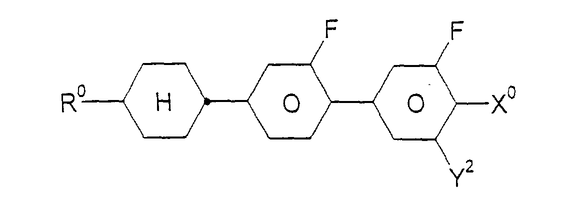 enthält,
worin
R H, einen unsubstituierten, einen einfach durch CN oder CF₃ oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S-, 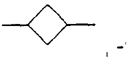 -CO-, -CO-O-, -O-CO- oder -O-CO-O- so ersetzt sein können, daß 0-Atome nicht direkt miteinander verknüpft sind,
Y F, Cl, halogeniertes Alkyl, Alkenyl oder Alkoxy mit 1 bis 6 C-Atomen, OCH = CF₂, OCF = CF₂, OCF = CFCF₃, OCF = CF-C₂F₅, CF₂OCF₃
L¹ H oder F
R⁰ n-Alkyl, Oxaalkyl, Fluoralkyl oder Alkenyl mit jeweils bis zu 7 C-Atomen,
X⁰ F, Cl, halogeniertes Alkyl, Alkenyl oder Alkoxy mit 1 bis 6 C-Atomen, OCH = CF₂, OCF = CF₂ und
Y² H oder F
bedeuten.

2. Medium nach Anspruch 1, **dadurch gekennzeichnet, daß** Y in Formel I, F, Cl, OCF₃, OCHF₂, CF₃, CHFCF₃, CF₂CHF₂, C₂H₄CHF₂, CF₂CH₂CF₃, CHF₂, OCH₂CF₃, OCH₂CHF₂, OCF₂CHF₂, O(CH₂)₃CF₃, OCH₂C₂F₅, OCH₂CF₂CHF₂, OCH₂C₃F₇, OCHFCF₃, OC₂F₅, OCF₂CHFCF₃, OCH=CF₂, OCF=CF₂, OCF=CFCF₃, OCF=CF-C₂F₅, CH=CHF, CH=CF₂, CF=CF₂, CF₂OCF₃ bedeutet.

3. Medium nach Anspruch 1, **dadurch gekennzeichnet, daß** es Verbindungen der Formel I enthält, worin Y = L¹ = F bedeutet.

4. Medium nach einem der Ansprüche 1 bis 3, **dadurch gekennzeichnet, daß** R in Formel I Ethyl, Propyl, Butyl, Pentyl, Hexyl, Heptyl, Ethoxy, Propoxy, Butoxy, Pentoxy, Hexoxy oder Heptoxy bedeutet.

5. Medium nach einem der Ansprüche 1 bis 3, **dadurch gekennzeichnet, daß** R in Formel I geradkettiges 2-Oxapropyl, 2- oder 3-Oxabutyl, 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl bedeutet.

6. Medium nach einem der Ansprüche 1 bis 3, **dadurch gekennzeichnet, daß** R in Formel I Vinyl, Prop-1- oder Prop-2-enyl, But-1-, 2- oder But-3-enyl, Pent-1, 2-, 3- oder Pent-4-enyl, Hex-1-, 2-, 3-, 4- oder Hex-5-enyl, Hept-1-, 2-, 3-,4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl bedeutet.

7. Medium nach einem der Ansprüche 1 bis 3, **dadurch gekennzeichnet, daß** R in Formel I Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy bedeutet.

8. Medium nach Anspruch 1, **dadurch gekennzeichnet, daß** das Medium zwei oder mehr Verbindungen der Formel I enthält.

9. Medium nach Anspruch 1, **dadurch gekennzeichnet, daß** es zusätzlich eine oder mehrere Verbindungen ausgewählt aus der Gruppe bestehend aus den allgemeinen Formeln II, III, IV, VI enthält: 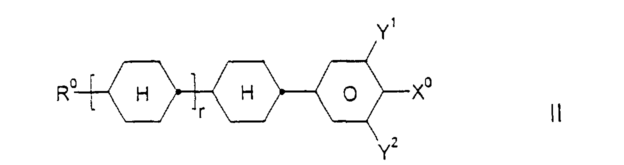 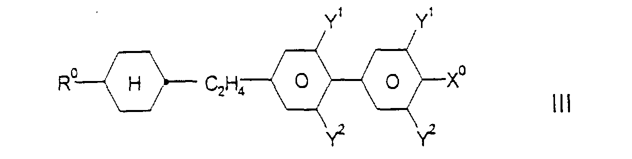 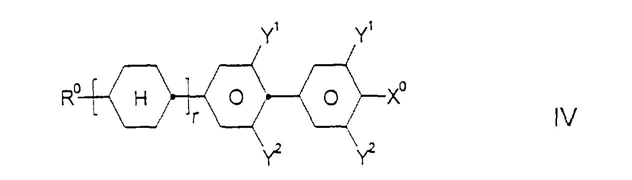 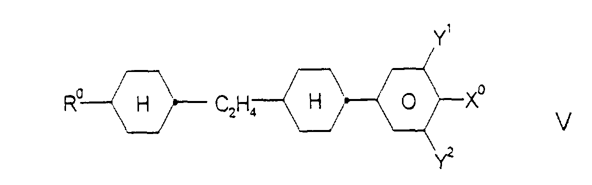 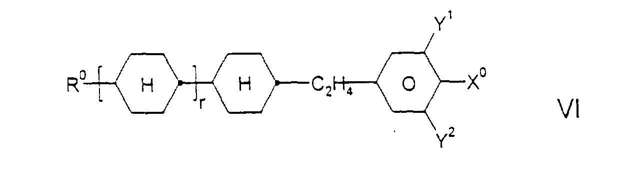 worin die einzelnen Reste die folgenden Bedeutungen haben:
R⁰ : n-Alkyl, Oxaalkyl, Fluoralkyl oder Alkenyl mit jeweils bis zu 7 C-Atomen,
X⁰: F, Cl, halogeniertes Alkyl, Alkenyl oder Alkoxy mit 1 bis 6 C-Atomen, OCH = CF₂, OCF = CF₂
Y¹ und Y²: jeweils unabhängig voneinander H oder F,
r: 0 oder 1.

10. Medium nach Anspruch 9, **dadurch gekennzeichnet, daß** 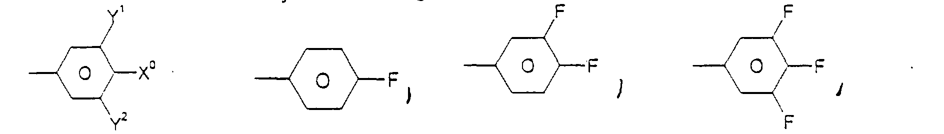 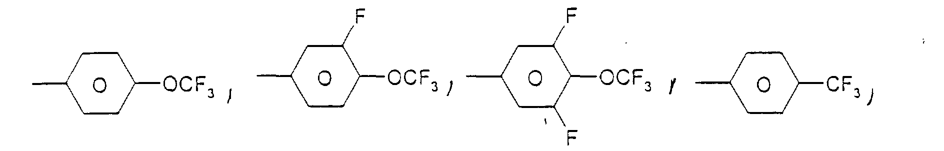 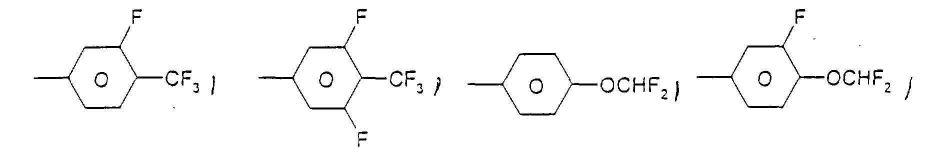 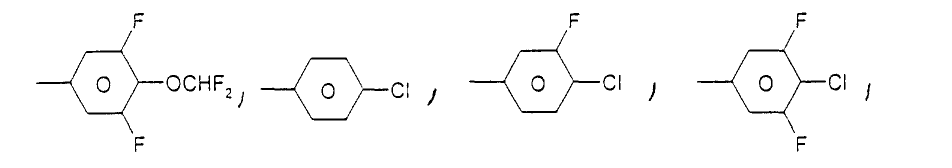 bedeutet.

11. Medium nach Anspruch 9, **dadurch gekennzeichnet, daß** X⁰ F, OCF₃, OCHF₂, OCH=CF₂, OCF=CF₂ oder OCF₂-CF₂H bedeutet.

12. Medium nach Anspruch 9, **dadurch gekennzeichnet, daß** der Anteil an Verbindungen der Formeln I bis VI zusammen im Gesamtgemisch mindestens 50 % Gew.% beträgt.

13. Medium nach Anspruch 1, **dadurch gekennzeichnet, daß** der Anteil an Verbindungen der Formel I im Gesamtgemisch 5 bis 95 Gew.% beträgt.

14. Medium nach Anspruch 9, **dadurch gekennzeichnet, daß** der Anteil an Verbindungen der Formeln II bis VI im Gesamtgemisch 20 bis 80 Gew.% beträgt.

15. Medium nach Anspruch 1, **dadurch gekennzeichnet, daß** X⁰ F oder OCF₃ und Y² H oder F bedeuten.

16. Medium nach Anspruch 1, **dadurch gekennzeichnet, daß** es zusätzlich ein oder mehrere Verbindungen der Formel 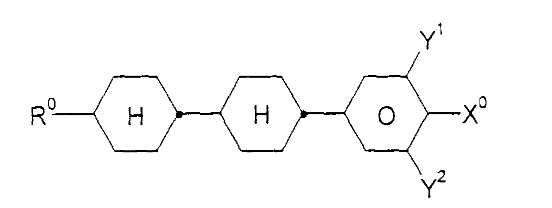 enthält, worin
X⁰ F₁ OCHF₂ oder OCF₃,
Y¹ und Y² jeweils unabhängig voneinander H oder F, und
R⁰ n-Alkyl, Oxaalkyl, Fluoralkyl oder Alkenyl mit jeweils bis zu 7 C-Atomen
bedeuten.

17. Medium nach Anspruch 1, **dadurch gekennzeichnet, daß** es zusätzlich ein oder mehrere Verbindungen der Formel XVII, 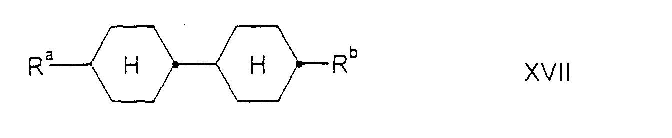 worin worin
R^{a} und R^{b} jeweils unabhängig voneinander geradkettiges Alkyl oder Alkoxy mit 1 bis 5 C-Atomen bedeuten,
enthält.

18. Verwendung des flüssigkristallinen Mediums nach Anspruch 1 für elektrooptische Zwecke.

19. Elektrooptische Flüssigkristallanzeige enthaltend ein flüssigkristallines Medium nach Anspruch 1.

## Claims

1. Liquid-crystalline medium based on a mixture of polar compounds of positive dielectric anisotropy, **characterized in that** it comprises one or more compounds of the general formula I 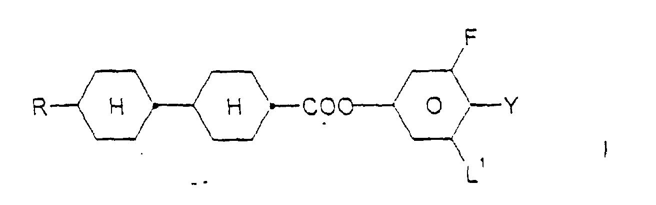 and additionally a compound of the formula 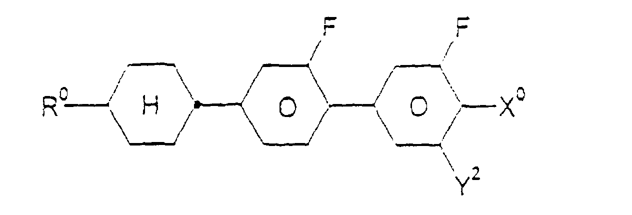 in which
R is H, an alkyl or alkenyl radical having 1 to 15 carbon atoms which is unsubstituted, monosubstituted by CN or CF₃ or at least monosubstituted by halogen, where, in addition, one or more CH₂ groups in these radicals may each, independently of one another, be replaced by -O-, -S-, 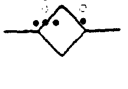 '-CO-, -CO-O-, -O-CO- or -O-CO-O- in such a way that O atoms are not linked directly to one another,
Y is F, Cl, halogenated alkyl, alkenyl or alkoxy having 1 to 6 carbon atoms, OCH=CF₂, OCF=CF₂, OCF=CFCF₃, OCF=CF-C₂F₅ or CF₂OCF₃,
L¹ is H or F,
R⁰ is n-alkyl, oxaalkyl, fluoroalkyl or alkenyl, each having up to 7 carbon atoms,
X⁰ is F, Cl, halogenated alkyl, alkenyl or alkoxy having 1 to 6 carbon atoms, OCH=CF₂ or OCF=CF₂, and
Y² is H or F.

2. Medium according to Claim 1, **characterized in that** Y in the formula I is F, Cl, OCF₃, OCHF₂, CF₃, CHFCF₃, CF₂CHF₂, C₂H₄CHF₂, CF₂CH₂CF₃, CHF₂, OCH₂CF₃, OCH₂CHF₂, OCF₂CHF₂, O(CH₂)₃CF₃, OCH₂C₂F₅, OCH₂CF₂CHF₂, OCH₂C₃F₇, OCHFCF₃, OC₂F₅, OCF₂CHFCF₃, OCH=CF₂, OCF=CF₂, OCF=CFCF₃, OCF=CF-C₂F₅, CH=CHF, CH=CF₂, CF=CF₂ or CF₂OCF₃.

3. Medium according to Claim 1, **characterized in that** it comprises compounds of the formula I in which Y = L¹ = F.

4. Medium according to one of Claims 1 to 3, **characterized in that** R in the formula I is ethyl, propyl, butyl, pentyl, hexyl, heptyl, ethoxy, propoxy, butoxy, pentoxy, hexoxy or heptoxy.

5. Medium according to one of Claims 1 to 3, **characterized in that** R in the formula I is straight-chain 2-oxapropyl, 2- or 3-oxabutyl, 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanonyl, or 2-, 3-, 4-, 5-, 6-, 7-, 8- or 9-oxadecyl.

6. Medium according to one of Claims 1 to 3, **characterized in that** R in the formula I is vinyl, prop-1- or -2-enyl, but-1-, -2- or -3-enyl, pent-1-, -2-, -3- or -4-enyl, hex-1-, -2-, -3-, -4- or -5-enyl, hept-1-, -2-, -3-, -4-, -5- or -6-enyl, oct-1-, -2-, -3-, -4-, -5-, -6- or -7-enyl, non-1-, -2-, -3-, -4-, -5-, -6-, -7- or -8-enyl, or dec-1-, -2-, -3-, -4-, -5-, -6-, -7-, -8- or -9-enyl.

7. Medium according to one of Claims 1 to 3, **characterized in that** R in the formula I is isopropyl, 2-butyl (= 1-methylpropyl), isobutyl (= 2-methylpropyl), 2-methylbutyl, isopentyl (= 3-methylbutyl), 2-methylpentyl, 3-methylpentyl, 2-ethylhexyl, 2-propylpentyl, isopropoxy, 2-methylpropoxy, 2-methylbutoxy, 3-methylbutoxy, 2-methylpentoxy, 3-methylpentoxy, 2-ethylhexoxy, 1-methylhexoxy or 1-methylheptoxy.

8. Medium according to Claim 1, **characterized in that** the medium comprises two or more compounds of the formula I.

9. Medium according to Claim 1, **characterized in that** it additionally comprises one or more compounds selected from the group consisting of the general formulae II, III, IV and VI: 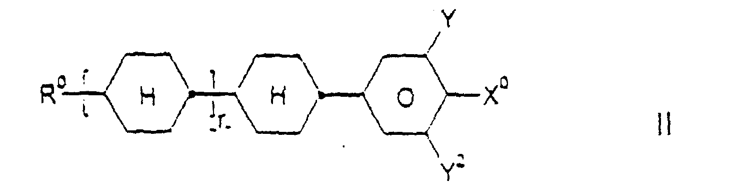 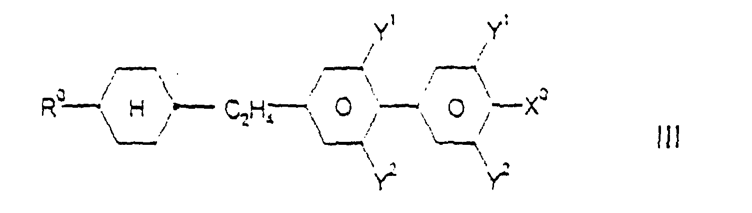 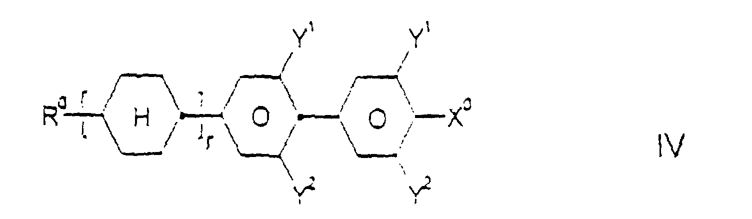 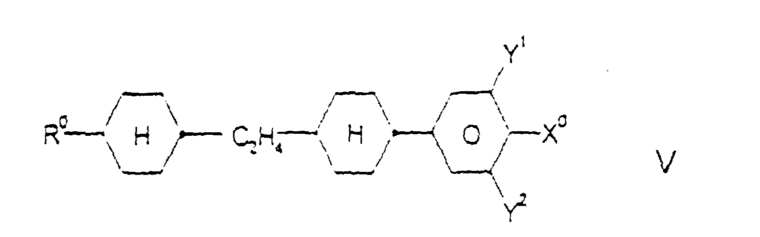 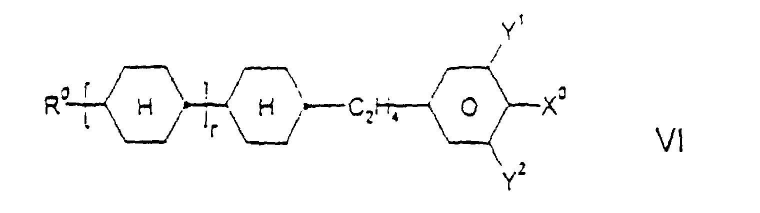 in which the individual radicals have the following meanings:
R⁰ : n-alkyl, oxaalkyl, fluoroalkyl or alkenyl, each having up to 7 carbon atoms,
X⁰ : F, Cl, halogenated alkyl, alkenyl or alkoxy having 1 to 6 carbon atoms, OCH=CF₂ or OCF=CF₂,
Y¹ and Y²: each, independently of one another, H or F,
r: 0 or 1.

10. Medium according to Claim 9, **characterized in that**

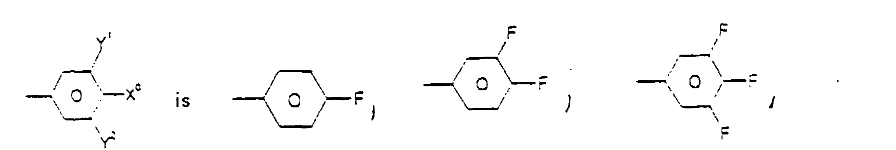

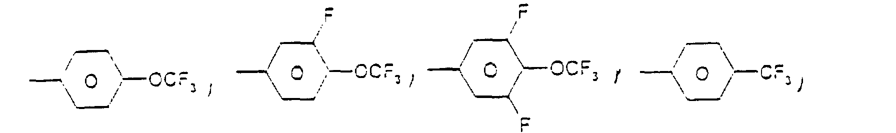

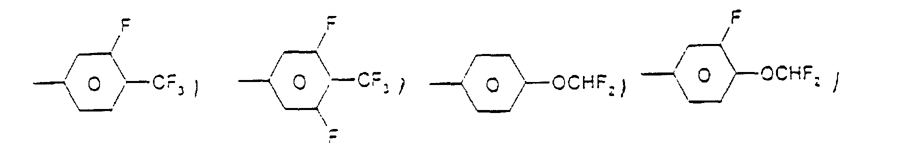

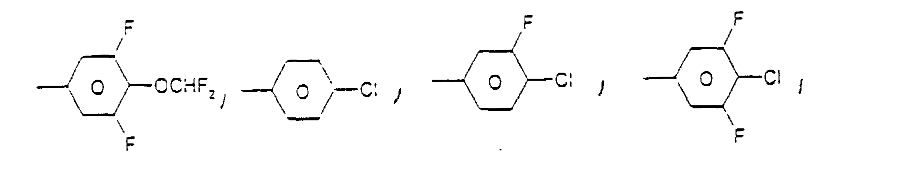

11. Medium according to Claim 9, **characterized in that** X⁰ is F, OCF₃, OCHF₂, OCH=CF₂, OCF=CF₂ or OCF₂-CF₂H.

12. Medium according to Claim 9, **characterized in that** the proportion of compounds of the formulae I to VI together in the mixture as a whole is at least 50% by weight.

13. Medium according to Claim 1, **characterized in that** the proportion of compounds of the formula I in the mixture as a whole is from 5 to 95% by weight.

14. Medium according to Claim 9, **characterized in that** the proportion of compounds of the formulae II to VI in the mixture as a whole is from 20 to 80% by weight.

15. Medium according to Claim 1, **characterized in that** X⁰ is F or OCF₃, and Y² is H or F.

16. Medium according to Claim 1, **characterized in that** it additionally comprises one or more compounds of the formula 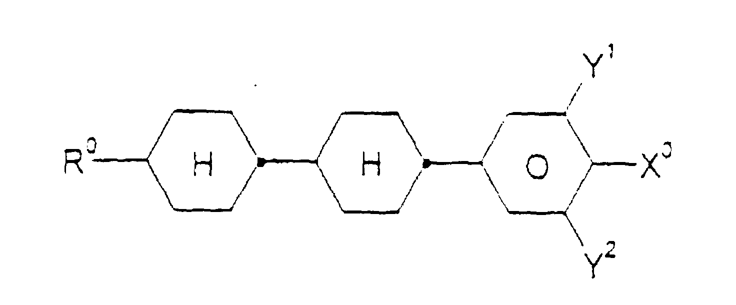 in which
X⁰ is F, OCHF₂ or OCF₃,
Y¹ and Y² are each, independently of one another, H or F, and
R⁰ is n-alkyl, oxaalkyl, fluoroalkyl or alkenyl, each having up to 7 carbon atoms.

17. Medium according to Claim 1, **characterized in that** it additionally comprises one or more compounds of the formula XVII 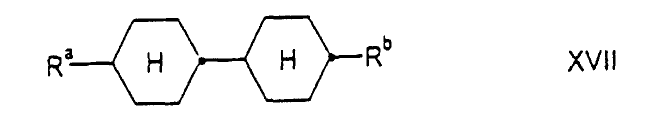 in which
R^{a} and R^{b} are each, independently of one another, straight-chain alkyl or alkoxy having 1 to 5 carbon atoms.

18. Use of the liquid-crystalline medium according to Claim 1 for electro-optical purposes.

19. Electro-optical liquid-crystal display containing a liquid-crystalline medium according to Claim 1.

## Revendications

1. Milieu cristallin liquide à base d'un mélange de composés polaires ayant une anisotropie diélectrique positive, **caractérisé en ce qu'**il contient un ou plusieurs composés de formule générale I : 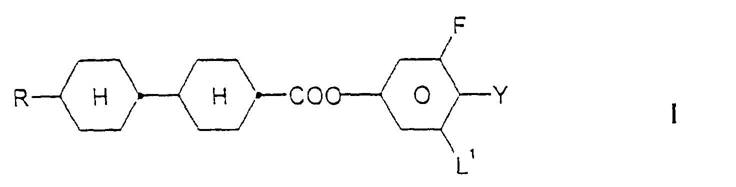 et en plus un composé de formule : 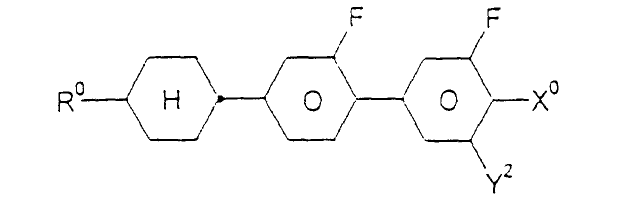 où
R représente H, un reste alkyle ou alcényle comportant 1 à 15 atomes de C, non substitué, monosubstitué par CN ou CF₃ ou au moins monosubstitué par un halogène, un ou plusieurs groupe CH₂ dans ces restes pouvant être remplacés, indépendamment les uns des autres, par -O-, -S-, 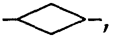 , -CO-, -CO-O-, -O-CO- ou -O-CO-O-, de telle façon que les atomes de O ne soient pas directement reliés entre eux,
Y représente F, Cl, un alkyle, un alcényle ou un alcoxy halogénés comportant 1 à 6 atomes de C, OCH=CF₂, OCF=CF₂, OCF=CFCF₃, OCF= CF-C2F5, CF₂OCF₃;
L¹ représente H ou F,
R⁰ représente un n-alkyle, un oxaalkyle, un fluoroalkyle ou un alcényle comportant jusqu'à 7 atomes de C,
X⁰ représente F, Cl, un alkyle, un alcényle ou un alcoxy halogénés comportant 1 à 6 atomes de C, OCH = CF2, OCF=CF₂ et
Y² représente H ou F.

2. Milieu selon la revendication 1, **caractérisé en ce que** Y représente dans la formule I F, Cl, OCF3, OCHF2, CF3, CHFCF3, CF2CHF2, C2H4CHF2, CF2CH2CF3, CHF2, OCH2CF3, OCH2CHF2, OCF2CHF2, O(CH2)3CF3, OCH2C2Fs, OCH2CF2CHF2, OCH2C3F7, OCHFCF3, OC2Fs, OCF2CHFCF3, OCH=CF2, OCF=CF2, OCF=CFCF3, OCF=CF-C2F5, CH=CHF, CH=CF2, CF=CF2, CF2OCF3.

3. Milieu selon la revendication 1, **caractérisé en ce qu'**il contient des composés de formule I où Y=L¹=F.

4. Milieu selon l'une des revendications 1 à 3, **caractérisé en ce que** R représente dans la formule 1 l'éthyle, le propyle, le butyle, le pentyle, l'hexyle, l'heptyle, l'époxy, le propoxy, le butoxy, le pentoxy, l'hexoxy ou l'heptoxy.

5. Milieu selon l'une des revendications 1 à 3, **caractérisé en ce que** R représente dans la formule I le 2-oxapropyle, le 2- ou le 3-oxabutyle, le 2-, le 3- ou le 4-oxapentyle, le 2-, le 3-, le 4- ou le 5-oxahexyle, le 2-, le 3-, le 4-, le 5- ou le 6-oxaheptyle, le 2-, le 3-, le 4-, le 5-, le 6- ou le 7-oxaoctyle, le 2-, le 3-, le 4-, le 5-, le 6-, le 7- ou le 8-oxanonyle, le 2-, le 3-, le 4-, le 5-, le 6-, le 7-, le 8- ou le 9-oxadécyle à chaîne droite.

6. Milieu selon l'une des revendications 1 à 3, **caractérisé en ce que** R représente dans la formule I, le vinyle, le prop-1- ou le prop-2-ényle, le but-1-, le but-2- ou le but-3-ényle, le pent-1-, le pent-2-, le pent-3- ou le pent-4-ényle, l'hex-1-, l'hex-2-, l'hex-3-, l'hex-4- ou l'hex-5-ényle, l'hept-1-, l'hept-2-, l'hept-3-, l'hept-4-, l'hept-5 ou l'hept-6-ényle, l'oct-1-, l'oct-2-, l'oct-3-, l'oct-4-, l'oct-5-, l'oct-6- ou l'oct-7-ényle, le non-1-, le non-2-, le non-3-, le non-4-, le non-5-, le non-6-, le non-7- ou le non-8-ényle, le dec-1-, le dec-2-, le dec-3-, le dec-4-, le dec-5-, le dec-6-, le dec-7-, le dec-8- ou le dec-9-ényle.

7. Milieu selon l'une des revendications 1 à 3, **caractérisé en ce que** R représente dans la formule I l'isopropyle, le 2-butyle (= le 1-méthylpropyle), l'isobutyle (= le 2-méthylpropyle), le 2-méthylbutyle, l'isopentyle (= le 3-méthylbutyle), le 2-méthylpentyle, le 3-méthylpentyle, le 2-éthylhexyle, le 2-propylpentyle, l'isopropoxy, le 2-méthylpropoxy, le 2-méthylbutoxy, le 3-méthylbutoxy, le 2-méthylpentoxy, le 3-méthylpentoxy, le 2-éthylhexoxy, le 1-méthylhexoxy, le 1-méthylheptoxy.

8. Milieu selon la revendication 1, **caractérisé en ce que** le milieu contient deux composés de formule I ou plus.

9. Milieu selon la revendication 1, **caractérisé en ce qu'**il contient en plus un ou plusieurs composés choisis dans le groupe constitué par les formules générales II, III, IV, V et VI : 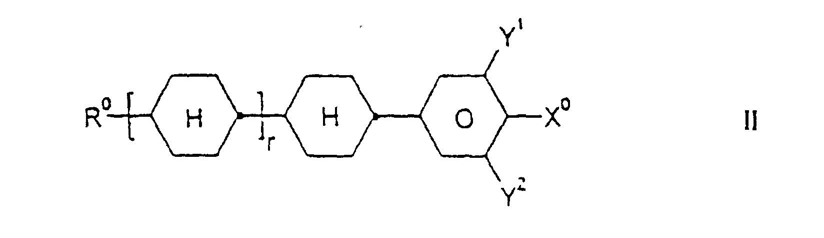 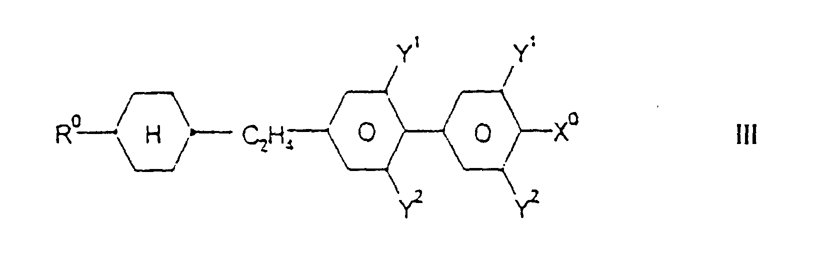 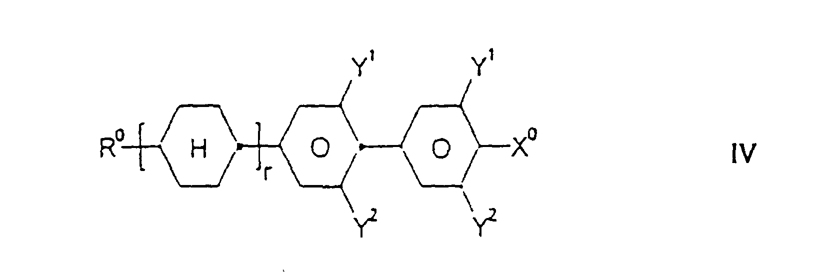 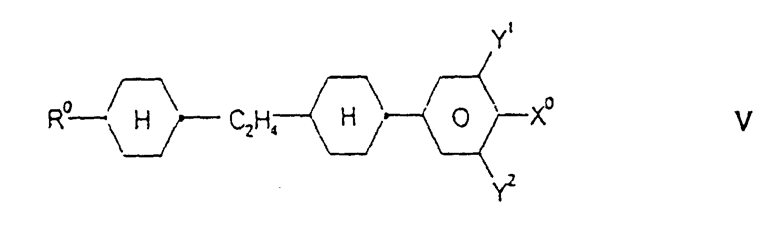 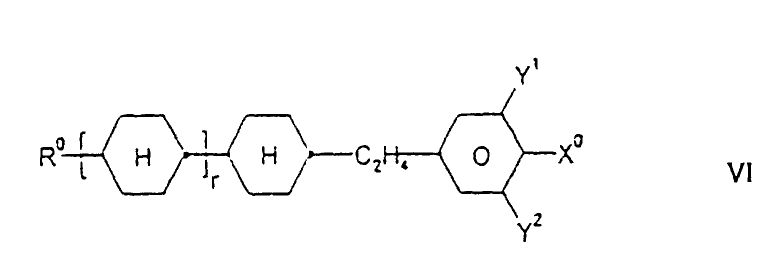 où les différents restes ont les significations suivantes :
R⁰ représente un n-alkyle, un oxaalkyle, un fluoroalkyle ou un alcényle comportant jusqu'à 7 atomes de C,
X⁰ représente F, Cl, un alkyle, un alcényle ou un alcoxy halogénés comportant 1 à 6 atomes de C, OCH=CF2, OCF=CF2,
Y¹ et Y² représentent, indépendamment l'un de l'autre, H ou F,
r est égal à 0 ou 1.

10. Milieu selon la revendication 9, **caractérisé en ce que** 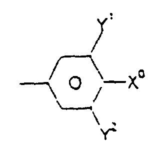 représente :

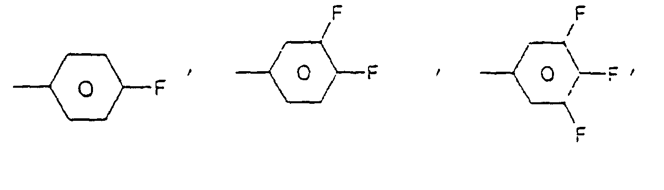

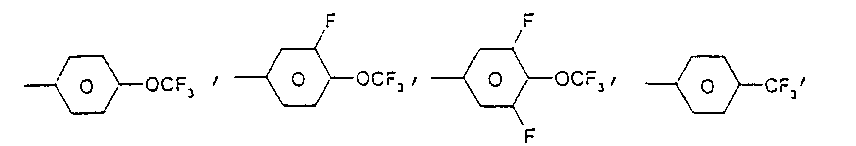

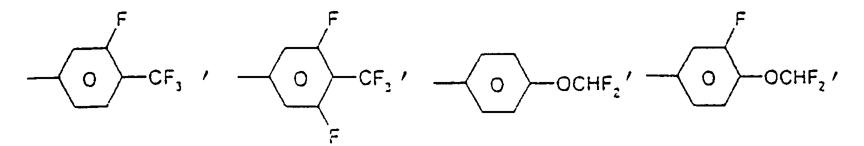

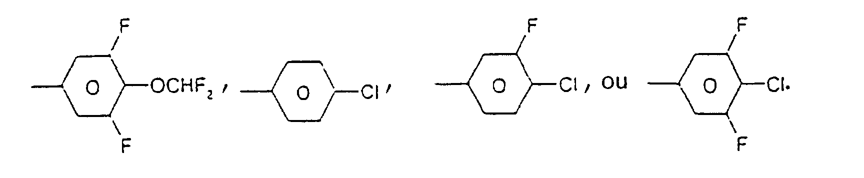

11. Milieu selon la revendication 9, **caractérisé en ce que** X° représente F, OCF₃, OCHF₂, OCH=CF2, OCF=CF₂ ou OCF2-CF2H.

12. Milieu selon la revendication 9, **caractérisé en ce que** la proportion en composés de formules I à VI dans le mélange total est d'au moins 50% en poids.

13. Milieu selon la revendication 1, **caractérisé en ce que** la proportion en composés de formule I dans le mélange total est comprise entre 5 et 95% en poids.

14. Milieu selon la revendication 9, **caractérisé en ce que** la proportion en composés de formules II à VI dans le mélange total est compris entre 20 et 80% en poids.

15. Milieu selon la revendication 1, **caractérisé en ce que** X⁰ représente F ou OCF3 et Y² représente H ou F.

16. Milieu selon la revendication 1, **caractérisé en ce qu'**il contient en plus un ou plusieurs composés de formule : 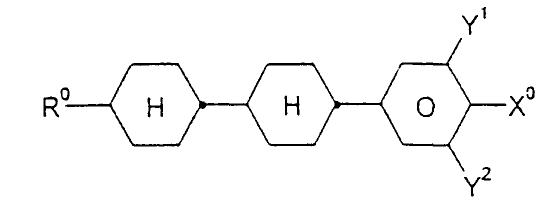 où
X⁰ représente F, OCHF2 ou OCF₃,
Y¹ et Y² représentent, indépendamment l'un de l'autre, H ou F, et
R⁰ représente un n-alkyle, un oxaalkyle, un fluoroalkyle ou un alcényle comportant jusqu'à 7 atomes de C.

17. Milieu selon la revendication 1, **caractérisé en ce qu'**il contient en plus un ou plusieurs composés de formule XVII : 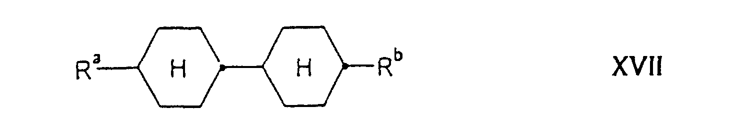 où
R^{a} et R^{b} représentent, indépendamment l'un de l'autre, un alkyle ou un alcoxy à chaîne droite comportant 1 à 5 atomes de C.

18. Utilisation du milieu cristallin liquide selon la revendication 1 à des fins électro-optiques.

19. Affichage à cristaux liquides électro-optique contenant un milieu cristallin liquide selon la revendication 1.
